# EUROPEAN PATENT APPLICATION

(11) **EP 0 684 050 A2**
(43) Date of publication of application: **29.11.1995**
(21) Application number: 95108030.8
(22) Date of filing: 24.05.1995
(51) Int. Cl.: A61M 39/00

(54) **Needleless injection site with bypass valve arrangement**

(30) Priority: 27.05.1994 US 250559
(71) Applicant: IVAC CORPORATION, San Diego, CA 92121 (US)
(72) Inventor: Daoud, Adib G., San Diego, California 92116 (US)
(74) Representative: Howden, Christopher Andrew

(57) **Abstract**

A needleless injection site allowing infusion and withdrawal of fluid therethrough is disclosed. When a compatible blunt male fluid connector fitting is inserted, fluid flow is accommodated; but upon withdrawal of the connector, the injection site is sealed against fluid flow. The injection site includes an elastomeric plunger having first and second ends and having an annular seal portion at a selected distance from the first end, movably contained within a fluid passageway through a housing having first and second ends, and biased towards a first position in sealing contact with the housing at the first end. The housing also has at least one fluid bypass opening into the passageway intermediate the ends a selected distance from the first end. Insertion of a compatible male fitting in the first end of the injection site moves the plunger to a second position where the annular seal portion of the plunger is located adjacent the fluid bypass opening. This allows fluid flow through the passageway, bypassing the seal portion through the fluid bypass.

## Description

### BACKGROUND OF THE INVENTION

### Field of the Invention

This invention generally relates to injection sites for use in the handling and administration of parenteral fluids. More particularly, the invention relates to an injection site configured to make sterile connections in IV or other fluid systems in medical applications without the use of a sharp cannula.

### Description of the Related Art

Injection sites for injecting or removing fluid from a system, such as an IV infusion set connected to a patient, or a fluid reservoir or drug vial, are well known and widely used. Conventional injection sites generally involve a pierceable septum formed of an elastomeric material, such as latex rubber or the like, captured in an access port. The housing of the access port may be, for example, the Y-body of a conventional Y-site component of an IV delivery set. A sharp sterile cannula is inserted into the access port, piercing the septum, and a distal end of the cannula is driven through and positioned distally in relation to the septum. In this way a fluid connection is made with the interior of the access port through the inserted cannula. Upon withdrawal of the cannula, the elastomeric septum reseals the puncture made by the cannula. Thus, a sterile environment can be maintained within the housing of the injection site. The outer surface of the septum of the injection site is wiped with an antiseptic before each use to prevent septic agents from being drawn into the access port by the piercing movement of the cannula.

Recently, connectors for accommodating the injection and withdrawal of fluids without the use of a sharp cannula have been used in increasing numbers instead of the above-described conventional injection sites. This is at least in part due to concern regarding the possible transmission of disease as a result of accidental needle punctures occasioned by handling the sharp cannula. Connectors having no sharpened surfaces are desirable because the chances of inadvertently piercing the skin are lessened.

However, some existing needleless injection sites allow fluid flow in only one direction, or require some further manipulation after a connection is established to allow fluid flow in both directions. These characteristics are undesirable because they either limit the usefulness of the connector or make it less convenient to use. A further concern in the design of needleless connectors is maintaining, as far as possible, a seal between the interior of the connectors to be connected and the atmosphere. For example, allowing fluid to escape or air to enter during connection due to the female connector being opened before the male connector is sufficiently seated are undesirable.

Additionally, it is desirable that needleless injection sites be configured so that they can be easily cleaned by an antiseptic wipe or similar means prior to making a connection. Optimally, exterior surfaces that may be involved in the transmission of fluid should be available for cleaning prior to the connection being made. Some prior connectors have small rifts or fissures, for example as may be defined by a clearance between parts, or formed adjacent an elastomeric element at the proximal end of the connector housing that is not under sufficient compression to form a tight seal with the housing. Such rifts or fissures may contain septic agents and are difficult to clean in attempting to sterilize the connector. Also, if a luer connector is employed, portions of the interior of a female luer connector well incorporated in an injection site may be difficult to reach to clean. A cap may be used to seal a female luer connector, however, injection sites requiring a cap to maintain a sterile connection port are undesirable because the extra steps of removing and replacing a cap are inconvenient for medical personnel.

Lastly, some existing needleless injection sites have a relatively complex configuration, and may also have a large number of component parts. Such connectors are relatively difficult and costly to manufacture and may have more problems in service, for example, such as sticking due to the difficulty of flushing medicaments from small dead spaces inherent in complex mechanism geometries.

Those concerned with the development of injection sites have recognized that along with a reduction in needlesticks, needleless connectors should provide for convenient connections, without undue manipulations of the connector being required. Removal and replacement of a cap which can become lost or contaminated should be avoided. Also, maintaining a seal during connection and during disconnection, the difficulty of sterilizing the connector prior to connection, reliability in use, and cost of manufacture are all aspects of needleless connector design where improvements over existing designs may yield beneficial results. Consequently, there is a continuing need, for a variety of reasons, for improvement in injection sites. The present invention addresses this need.

### SUMMARY OF THE INVENTION

Briefly, and in general terms, the injection site of the invention includes a housing having a first, or proximal end and a second, or distal, end, an interior portion of the housing defining a passageway therebetween allowing fluid communication between first and second ends, the first end defining a connector port. Also incorporated in the housing is at least one fluid bypass opening into the passageway intermediate the ends of the passageway a selected distance from the first end. An elastomeric plunger, having a first end, a second end and an annular seal portion, is movably disposed within the passageway. The plunger has a first, closed position, and a second, open position to which it can be moved, for example by insertion of a compatible blunt male connector fitting into the first end defining a connection port of the injection site housing. A biasing member is provided within the passageway, and biases the plunger to the first position. Movement of the plunger to the second position is against the force exerted by the biasing member.

With the plunger at its second position, the annular seal portion is located adjacent the fluid bypass opening. Fluid passes by the annular seal portion by flowing from the passageway into the fluid bypass, and around the annular seal portion of the plunger, and then back into the passageway on the opposite side of the annular seal portion.

When the plunger is returned to its first position, for example by removing an inserted male fitting, the injection site is again sealed. The configuration has the advantage of fully opening to allow full flow only when a male connector is deeply inserted or seated, which results in minimization of leakage during connection and disconnection.

Preferably the opening of the fluid bypass is located in said passageway and is of such length along the passageway in a direction from the first end to the second end of said housing, that in cooperation with the selected size of the connection port the injection site will accept male luer fittings having diameter and taper parameters within the predetermined ranges where said ranges define commonly used male luer fitting sizes.

The needleless injection site in a more detailed aspect can incorporate a further annular seal portion disposed about the first end of the plunger, which in the closed first position of the plunger is flush with the proximal end of the injection site housing. This second seal effectively seals the interior of the female connector well from contamination at the proximal or first end of the injection site. Also, due to the provision of the further seal at the first end of the female connection port and at the proximal end of the housing, the injection site is easily wipeable, and a separate protective cap is not required.

The further annular seal portion when provided at the first end of the plunger also requires a bypass, and one is provided in the passageway so that the seal at the first end of the plunger is adjacent a fluid bypass incorporated in the passageway wall when the plunger is moved to the second position. Again, the opening of the further bypass is placed a selected distance from the first end so that the seal portion at the first end of the plunger does not move into position to open a fluid pathway through the further bypass channel until a compatible blunt male connector fitting is deeply inserted or seated into the injection site. This order of occurrences also acts to prevent fluid or air from entering or escaping from the injection site during insertion or withdrawal of a connector in conjunction with the similar interactions of the first annular seal portion and the fluid bypass channel.

The plunger is urged towards sealing contact with the housing, which occurs at the first position adjacent the first end of the housing, by the biasing member. The biasing member is positioned between the plunger and the second end of the housing within the passageway therethrough. In a more detailed aspect, the biasing member is supported by raised ribs incorporated in the housing which also provide a fluid flow clearance around an end of the biasing member adjacent the second end of the housing. These raised ribs give rise to improved fluid flow through the injection site by providing a fluid path around the outside of the biasing member and allow the use of a biasing member that does not permit fluid flow therethrough, such as a solid elastomeric extension of the elastomeric plunger for example.

The plunger and the housing can each be provided with frustoconical portions which make the plunger self-seating in sealing contact with the housing in the first position due to the urging of the biasing member, resulting in improved reliability for the injection site. Particularly, the annular seal portion distal of the first end of the plunger is given a frustoconical configuration, which interacts with a corresponding frustoconical seat portion of the housing.

In a further more detailed aspect, a plurality of bypass openings can be employed, disposed circumferentially around the interior passageway of the housing. This configuration is advantageous in it results in more fluid flow capacity through the injection site.

In applications where the injection site is incorporated in a Y-site, a side branch port opening of an intersecting fluid passageway (and a portion of the intersecting passageway itself) inherent in the Y-site geometry can constitute a bypass channel. This latter configuration is advantageous because the injection site geometry is simplified.

According to another aspect of the invention there is provided an injection site adapted to receive a blunt male fluid connector fitting having diameter and taper parameters within selected ranges, to facilitate infusion and withdrawal of fluid through the injection site without use of a sharp cannula, comprising:
a housing having a first end defining a female connection port and a distal end and a passageway therebetween allowing fluid communication between the first end and second ends, a fluid bypass defined by a fluid pathway of selected length disposed radially outward of the inner walls of the housing defining said passageway and opening into the passageway intermediate the ends of the housing a selected distance from the first end, and a further fluid bypass defined by a fluid pathway incorporated in said female connection port disposed radially outward from an inside surface of said connection port and opening into the connection port a selected distance from said first end;
an elastomeric plunger, having a first end and a second end, movably disposed within said housing, the first end thereof being configured to provide a fluid flow pathway between the plunger and an inserted blunt male cannula, said plunger having an annular seal portion at the first end, and an annular seal portion intermediate the first and second ends, and a first position in sealing contact with the housing at the first end thereof, and a second position within the passageway of said housing at which the annular seal portion the first end of the plunger is located adjacent the fluid bypass in the connector port at the first end of the housing and the annular seal portion located between the first end and the second end of the plunger is located adjacent the fluid bypass opening located between the first and second ends of the housing;
biasing means for biasing the plunger to said first position at the first end of said housing and into sealing contact therewith; whereby fluid flow through the injection site is prevented when the plunger is in said first position and the first end of said housing defining a female connection port is sealed against contamination by the annular seal portion at the first end of the plunger, and fluid is allowed to flow through said injection site when the plunger is in said second position, fluid passing by the annular seal portion at the first end of the plunger by flowing into the fluid bypass incorporated in the wall of the connector port at the first end of the housing and around the annular seal portion of said plunger at the first end thereof, and fluid passing by the annular seal portion of the plunger disposed between the first and second ends of the plunger by flowing into the fluid bypass embodied in the wall of the passageway between the first and second ends of the housing and around this annular seal portion of the plunger disposed between the first and second ends thereof, the distance from the first end of the housing to each of the fluid bypass openings and the diameter of the connection port being chosen so as to allow the annular seal portions of said plunger to be adjacent the respective bypass openings when a male blunt cannula having diameter and taper parameters within said selected ranges is inserted.

Preferably the biasing means comprises a coil spring and the injection site further comprises ribs formed in the housing carrying the spring and providing a clearance between the spring and the housing, allowing increased fluid flow around the bottom of said spring.

Preferably at least one annular seal portion of the plunger comprises a frustoconical surface and the passageway through the housing member has an increasing diameter portion wherein the diameter increases with distance from the first end, and at least one annular seal portion of said plunger is located within said increasing diameter portion. In this case, the site preferably comprises a fluid bypass channel extending toward the connector port at the first end of the injection site beyond the side branch port, which allows fluid to bypass the annular seal portion at the second end of the plunger when said annular seal is not adjacent the side branch port.

Preferably, in this case, the further annular seal portion disposed about the first end of said plunger is formed of two annular raised portions position in close proximity to each other, forming a double annular seal at the first end of said plunger.

The needleless injection site may further comprise a check valve disposed within and supported by the side branch passageway of the Y-site to prevent retrograde flow through the Y-site from one branch thereof out through the side branch having the check valve therein.

According to a further aspect of the invention, there is provided a Y-site adapted to receive a blunt male fluid connector fitting to facilitate injection and withdrawal of fluid through said Y-site, and which seals upon removal of said male fitting, comprising:
a housing having a first end and a second end and a passageway therebetween allowing fluid communication between the first end and the second ends, and a fluid bypass comprising a side branch port opening into the passageway intermediate the ends and a portion of a side branch fluid passage, said housing defining a female connector at the first end, and having a further fluid bypass channel embodied in said female connector, and raised ribs disposed radially about the interior of said passageway adjacent the second end thereof allowing fluid flow between said ribs into a central portion of the passageway defining an outlet;
an elastomeric plunger, having a first end and a second end, movably disposed within said housing, having a raised portion at the first end thereof to provide a fluid flow clearance between said plunger and an inserted male fitting, and having an annular seal portion at the first end thereof and another annular seal portion between the first and second ends thereof, said plunger cooperating with said housing so that insertion of a male fitting in the first end of the housing displaces the plunger from a first position sealingly contacting said housing at the first end thereof, to a second position where an annular seal portion is located adjacent said fluid bypass comprising the side branch port, and the annular seal portion at the first end of the plunger is located adjacent the fluid bypass within said female connector portion;
biasing means for biasing said plunger to said first position in sealing contact with the housing at the first end thereof, the biasing means being carried by said raised ribs formed in the housing adjacent the second end thereof; whereby, when said plunger is in said first position, fluid flow through said female connector is prevented and the first end of said housing is sealed against contamination by the annular seal portion at the first end of the plunger, and, when said plunger is in said second position, fluid flow through said female luer connector is accommodated, fluid passing by the annular seal portion of the plunger positioned adjacent the side branch port comprising the fluid bypass by flowing into said side branch port and around said annular seal portion of the plunger, and fluid passing through the female fluid connector passing by the annular seal portion at the first end of the plunger by flowing into the fluid bypass disposed in the female connector portion and around the annular seal portion at the first end of said plunger, the dimensions of the plunger and the female connection port at the first end of the housing and the distances from the first end of the housing to the respective fluid bypass openings being selected so that the annular seal portions of the plunger will be adjacent the respective bypass openings over a range of dimensions defining male fittings commonly used define the male fitting inserted.

The needleless Y-site may further comprise a fluid bypass channel formed in the housing which extends beyond the side branch port towards the connector port at the first end of the Y-site, allowing fluid to bypass the annular seal portion of the plunger at the second end of the plunger through said fluid bypass channel before said annular seal is positioned in the passageway adjacent the side branch port as the plunger is moved into the injection site.

The passageway through the housing may have an increasing diameter portion wherein the diameter increases with distance from the first end of the housing, and the annular seal portion positioned between the first and second ends of said plunger is located within said increasing diameter portion.

The Y-site may further comprise a check valve disposed in and carried by said side branch passageway, preventing retrograde flow from the passageway disposed between the first and second ends of the injection site out through the side branch.

These and other features and advantages of the invention will become more apparent from the following detailed description, taken in conjunction with the accompanying exemplary drawings.

### BRIEF DESCRIPTION OF THE DRAWINGS

FIGURE 1 is a perspective view of an injection site configured in accordance with the invention;
FIG. 2 is an exploded perspective view of the injection site shown in FIG. 1;
FIG. 2a is a more detailed elevational view of a portion of the injection site shown in FIG. 1;
FIG. 3 is a longitudinal cross-section view of the injection site shown in FIG. 1 with the plunger in a first position taken along lines 3-3 in FIG. 1;
FIG. 4 is a longitudinal cross-section view of the injection site shown in FIG. 1 showing a male fitting connected, and the plunger in a second position;
FIG. 5 is a medial cross-section view of a bypass channel portion of the injection site shown in FIG. 1, taken along lines 5-5 in FIG. 4;
FIG. 6 is a medial cross-section view of a bypass channel portion of an injection site in accordance with the invention, taken along lines 6-6 in FIG. 7;
FIG. 7 is a longitudinal cross-section view of an alternate embodiment of an injection site according to the invention showing the plunger in a first position;
FIG. 8 is a cross-section view of the injection site of FIG. 7 showing a male fitting connected and the plunger in a second position;
FIG. 9 is a longitudinal cross-section view of a proximal connector port portion of a housing of an alternate embodiment of the injection site of the invention;
FIG. 10 is an end view, taken along line 10-10 in figure 9 of the connector port shown in FIG. 9;
FIG. 11 is a longitudinal cross-section view of an alternate embodiment of an injection site according to the invention;
FIG. 12 is a medial cross-section view of the injection site of FIG. 11 taken along line 12-12 in FIG. 11; and
FIG. 13 is a detailed elevational view of the plunger of the injection site of FIG. 11.

### DETAILED DESCRIPTION OF THE PREFERRED EMBODIMENTS

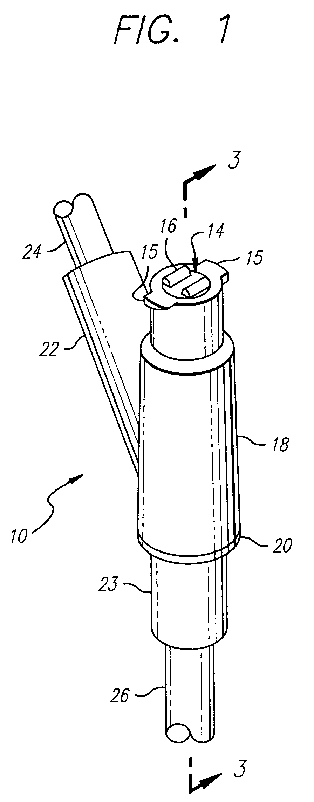

As shown in FIGURE 1 for purposes of illustration, the invention is embodied in a fluid injection site 10 of the type generally known in the art as a "needleless" connector, incorporated in a Y-site. The injection site is capable of receiving a compatible male luer connector fitting (not shown), with or without a luer lock, in a female luer connection port portion 14, displacing an elastomeric plunger 16. A luer lock portion 15 is unitary with the housing at the proximal connection port portion.

When no male connector is inserted, the elastomeric plunger 16 is at a first position at the first or proximal end of the injection site 10, flush with the connection port portion 14. The well of the female connection port is sealed by the elastomeric plunger. and there is no opportunity for fluid to pool in the exterior geometry of the connector. This configuration also allows the injection site to be easily wiped, for example by an alcohol (not shown), to sterilize the proximal end of the injection site. These features reduce the chance that infectious agents will be introduced into the interior of the injection site when a male connector is inserted.

A housing of the injection site 10 is formed of two members, a first or proximal housing member 18 and a second or distal housing member 20. The proximal housing member embodies a tubing connector Y-branch 22 for connection to a flexible tubing line 24. The second housing member 20 incorporates a connector portion 23 for connection to another flexible tubing line 26. The flexible tubing lines 24, 26 are typically part of a fluid system, for example an IV administration set (not shown), connected through a venipuncture site to the circulatory system of a patient.

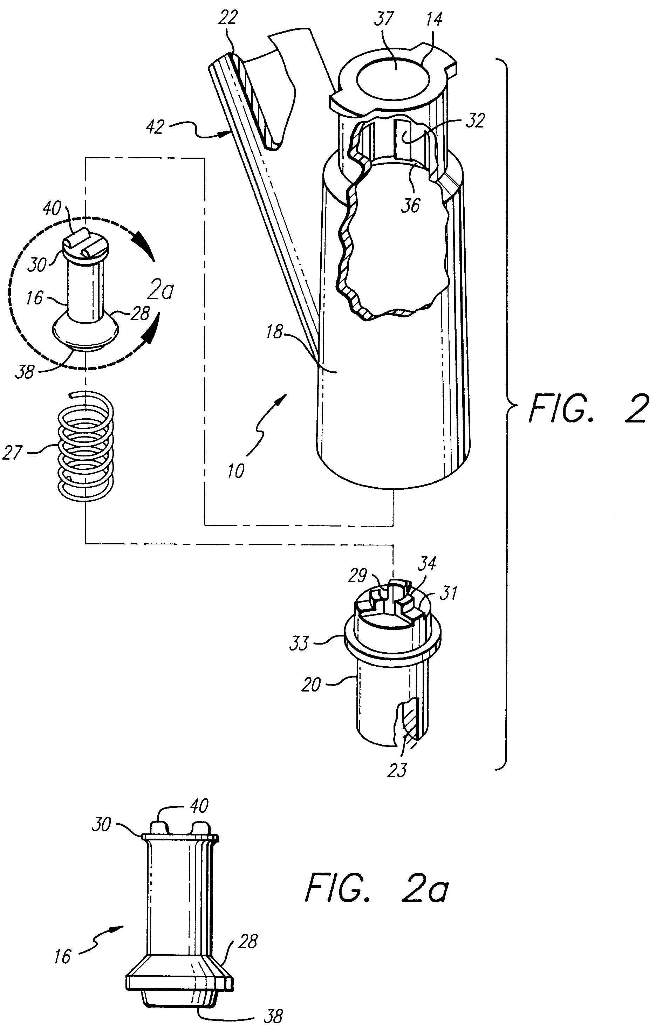

Turning to FIG. 2, in the illustrated embodiment the needleless connector 10 is comprised of four parts, two of which make up the housing, 18 and 20. The simplicity of the design can be appreciated with reference to this illustration, and advantages in terms of reliability, ease of manufacture and use, and cost will be apparent from the discussion herein. During assembly, the first or proximal housing member 18 receives the elastomeric plunger 16, which is self-seating. A stainless steel coil spring 27 is also inserted, which urges the elastomeric plunger towards the first end of the housing and into the female luer port 14. The spring 27 is confined and compressed by insertion of the second housing member 20.

In a currently preferred embodiment, the housing members 18, 20 are formed of a hard, resilient thermoplastic material, such as a polycarbonate resin, or a polyurethane, such as Isoplast, to name two examples. The second or distal housing member 20 incorporates an annular stop ring 33 that interacts with the first housing member to limit insertion of the second housing member and provide alignment of the two housing members to simplify assembly and help provide a sealed connection between the two housing members. The sealed connection can be made by conventional methods of bonding, for example ultrasonic or solvent welding. As will be apparent to one skilled in the art, the two housing portions could also be made of differing materials.

The second housing member 20 incorporates an outlet 29 in fluid communication with the tubing connector portion 23. The second housing member also includes raised radially disposed rib portions 31 located at three locations spaced apart by 120° about the outlet. These raised rib portions provide clearance for fluid flow around the distal end of the coil spring. In the illustrated embodiment each raised portion 31 also incorporates a further raised centering portion 34, that engages the inside of the coil spring and acts to center the distal end of the spring within the joined housing members 18, 20. These features provide improved functioning and reliability of the injection site by helping to keep the spring 27 in alignment with a central axis of the injection site and the inlet port 14, and by improving fluid flow through the injection site around the distal end of the coil spring.

In a currently preferred embodiment, the plunger 16 is deformable, and is made of a thermoplastic elastomer, for example, including materials such as silicone, or Kraton. The plunger includes a annular seal portion 28 that is frustoconical, reliably forming a seal when urged into contact with a corresponding frustoconical seat portion 36 of the proximal housing member 18 in the assembled injection site by the coil spring biasing member 27. This annular seal portion is disposed about a distal portion of the plunger, adjacent the second end of the plunger. The plunger also incorporates a raised centering portion 38 at its second or distal end, to interfit with the central opening defining the spring coils at the proximal end of the spring to aid in maintaining alignment of the plunger and spring.

Further, to provide a seal against leakage, and to guard against the introduction of contaminants into the female luer connection port 14, the elastomeric plunger 16 incorporates a further annular seal portion 30 at its first or proximal end. Also, at least one raised portion 40 is provided at the first end, which acts to hold the first end of the plunger away from an inserted male luer fitting (not shown). The raised flow clearance portion and forms a fluid flow pathway that allows fluid to flow past the plunger and in or out of the tip of such an inserted male connector in spite of the forces pushing the tip of the compatible connector and the first end of the plunger together when the tip pushes the plunger into the connecter port 14. Such a circumstance would otherwise make infusion of fluid difficult and withdrawal nearly impossible. In the illustrated embodiment two raised flow clearance portions 40 are provided.

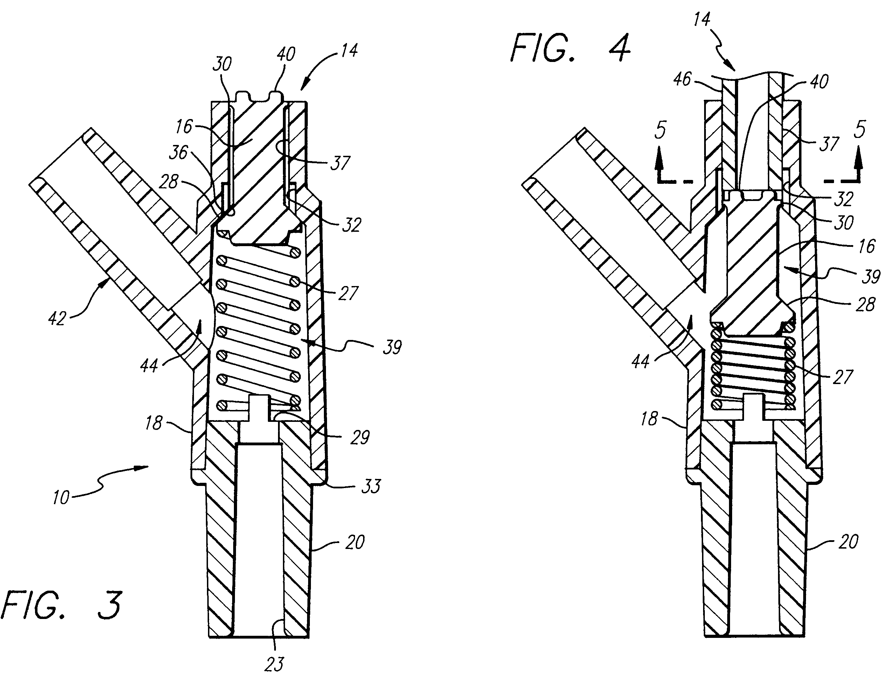

With reference to FIGS. 2 and 3, in the illustrated embodiment the first or proximal housing member 18 incorporates at least one fluid bypass 32 in a distal portion of the female luer connector well 37 of the female luer connector port 14. Six such bypass channels are included in the illustrated injection site. These bypass channels allow fluid to flow around the annular seal portion 30 at the first end of the elastomeric plunger 16 when a male luer fitting (not shown) is inserted and pushes the plunger into the connector port 14. This will be further discussed below in connection with the other drawing figures.

The illustrated proximal housing member 18 also incorporates a side branch 42 having a tubing connector portion 22 as previously described. Referring to FIG. 3, a side branch port 44 is defined by the proximal housing member 18 where the side branch intersects with a passageway 39 through the injection site 10. The passageway extends from the female luer connection port 14 at the first or proximal end of the injection site to the outlet 29 which in turn opens into the distal tubing connector 23 at the second or distal end of the injection site. The side branch port and intersecting side branch constitute a fluid bypass to the passageway as will be discussed below.

As can also be appreciated with reference to FIG. 3, the elastomeric plunger 16 is at a first position within the passageway 39 when an assembled injection site 10 is in an unconnected or closed state. In this first position, annular seal portion 28 of the elastomeric plunger 16 is seated against the frustoconical seat portion 36 of the proximal housing member 18 due to the urging of the spring 27, preventing flow through the female luer connection port 14. The further annular seal portion 30 disposed about the proximal end of the plunger 16 is positioned at the proximal end of the luer well 37. As can be appreciated, the well of the female luer connection port is sealed against contamination by the further seal at the proximal end of the plunger. This sealing feature eliminates the need for a protective cap, and also facilitates wiping of the proximal end of the injection site for sterilization purposes as the further seal is flush with the first end of the injection site 10 when the plunger is in its first position..

Turning to FIG. 4, a blunt connector fitting, such as a male luer fitting 46, is shown inserted into the female luer connection port 14. The elastomeric plunger 16 is moved by insertion of the male luer fitting to a second position within the passageway 39. Raised portions 40 hold the male luer fitting 46 away from the plunger 16, and provide a clearance for fluid flow in and out of the male fitting tip between the fitting and plunger when the male luer fitting is inserted into the injection site. In this second position of the plunger 16, the annular seal portion 28 is adjacent the side branch port 44. As mentioned, the side branch port performs the function of a first fluid bypass pathway intermediate the first and second ends of the injection site, allowing fluid to pass by the annular seal portion by flowing into the side branch port and around the annular seal portion and back into the passageway on the opposite side of the annular seal portion. The distance from the first of proximal end of the housing to the side branch port is selected in conjunction with the diameter of the connector port 14 to provide for connection of common sizes of male luer fittings.

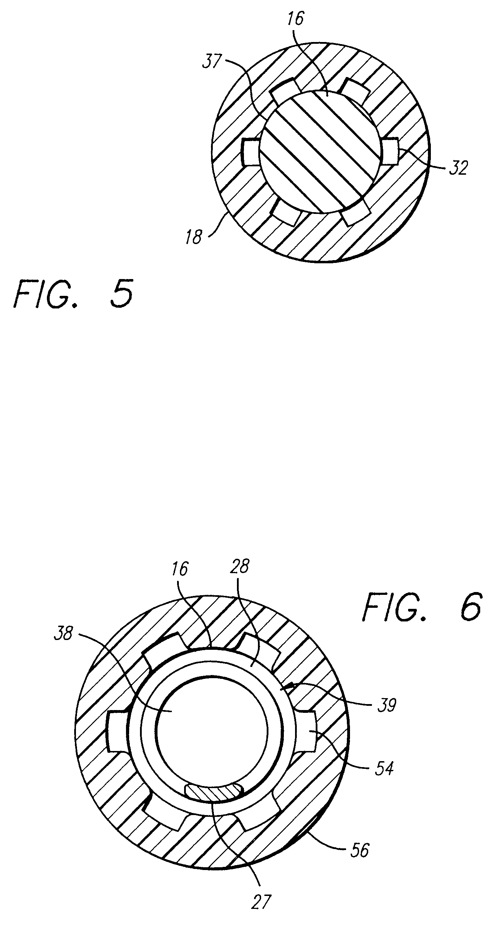

With the plunger 16 in this second position within the passageway 39, the further annular seal portion 30 at the first end of the plunger is positioned adjacent one or more further fluid bypass openings 32 located in a distal portion of the well 37 of the female luer connection port 14. Placement of the proximal annular seal 30 adjacent the bypass openings 32 there allows fluid to flow around the annular seal portion 30 at the first end of the plunger through such further bypass channels. The distance from the proximal end of their connector 14 and the length of these further fluid bypass openings 32, which in the illustrated embodiments are in the form of grooves or channels in a distal portion of the wall of the connector well 37, are chosen so that the annular seal portion 30 at the proximal end of the plunger 16 will be adjacent the fluid bypass 32 for a variety of common sizes of luer fitting 46 that may be inserted. Referring to FIG. 5, in the illustrated embodiment six such bypass grooves or channels 32 opening into the luer well portion 37 of the passageway are provided.

Returning to FIG. 4, it will be appreciated that the second position of the plunger 16 can vary, depending on the depth of penetration of the male luer connector 46. As will be apparent to one skilled in the art, this depth will depend on the relative size and taper of the inserted male luer fitting compared with that of the female luer connection port 14. As mentioned, the dimensions of the various members and the distances from the first end of the housing 18 to the fluid bypass openings 44,32 are chosen so that the annular seal portions 28,30 of the plunger 16 will be adjacent the respective fluid bypass openings 44, 32 as discussed above for a range of common male luer fitting sizes.

For example, it has been found that in conjunction with a female connector well 37 having a diameter between about 0.168 inches and about 0.170 inches, and a luer taper of about .060 in/in on diameter, the distance to the fluid bypass 32 within the distal portion of the female connector well 37 may be about 0.187 inches. The distance from the first end of the housing 18 to a fluid bypass opening 44 within the passageway formed by the side branch port may be for example about 0.187 inches plus the distance between annular seals disposed about the plunger. In this example, the distance between seals could be chosen to be about .333 inches, resulting in a distance from the first end of the housing to the bypass 44 of about 0.52 inches for a female connector opening 37 of this size.

Additionally, in the illustrated embodiment a very slight draft, or taper, is incorporated in the proximal housing member 18 defining the passageway 39. Thus, when a male luer 46 is being inserted, the clearance allowing fluid flow around the distal annular seal portion 28 of the plunger 16 within the passageway will widen with increased depth of penetration. Consequently, even if the distal annular seal portion is not directly adjacent the side branch opening 44, a small amount of fluid flow around the distal annular seal portion is possible, albeit with more effort than if the distal annular seal portion 28 of the plunger were positioned adjacent the side branch port 44 providing a fluid bypass. As a significant amount of fluid pressure is necessary to overcome the pressure loss associated with forcing a usable quantity of fluid through the small annular clearance however.

As will be apparent to one skilled in the art, flow through the connection port 14 is restricted by the proximity of the distal annular seal portion 28 of the plunger 16 to the wall of the proximal housing member 18 unless the distal annular seal portion is adjacent the side branch port 44, and also unless the annular seal portion 30 at the first end of the plunger 16 is adjacent a fluid bypass 32 in the luer well 37. This gives rise to the result that the injection site 10 will remain effectively closed to fluid flow until an inserted male luer fitting 46 is deep into the well 37 of the female connection port 14, forming contact seal between the male luer fitting and the well 37 of the female luer connector port 14. In turn this has the effect of greatly reducing leakage of fluid or air during the connection or disconnection process.

When a male luer fitting 46 is connected, it is held in the female luer connection port 14 of the injection site 10 by a friction fit, or by means of luer lock fittings (not shown). As will be apparent, fluid flow in either direction through the injection site is accommodated as long as a male luer fitting is connected, maintaining the plunger 16 at the second position described above.

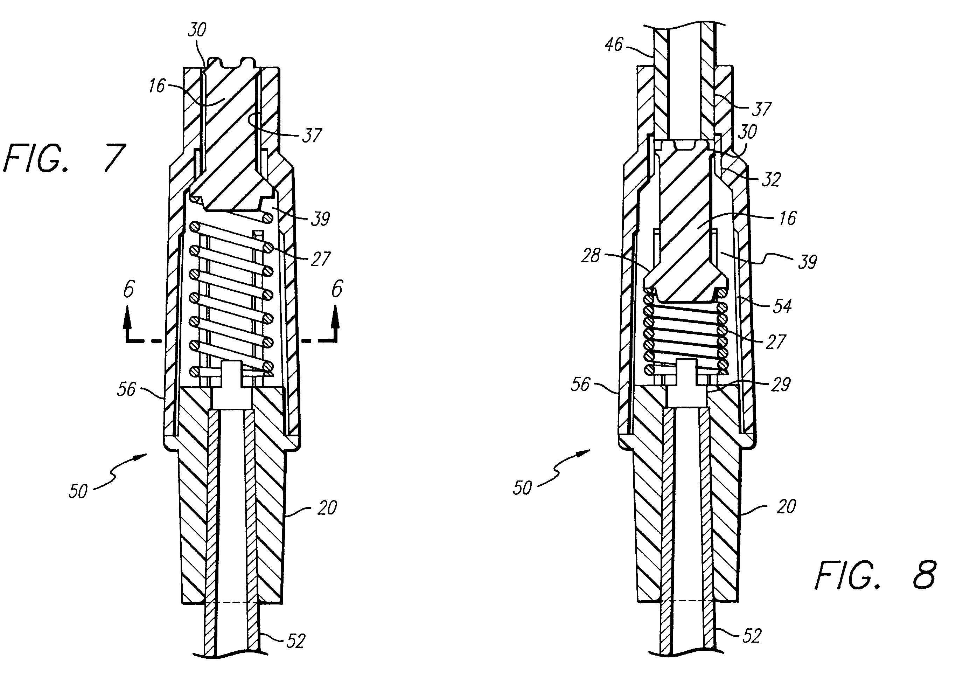

An alternate embodiment is shown in FIGS. 6,7 and 8, wherein a single injection site 50 according to the invention is connected to an end of a IV tubing line 52. The operation of the needleless injection site 50 is essentially the same as that previously described. However, since there is no side branch, as found in a Y-site, a different fluid bypass arrangement is provided. Referring to FIGS. 6 and 7, at least one bypass channel 54 is provided in a first or proximal housing member 56. As can be seen in figure 6, six such channels are provided in the illustrated embodiment. Referring to FIG. 7, the fluid bypass opens into passageway 39 through the housing to allow fluid to flow around the distal annular seal portion 28 of the elastomeric plunger 16 when the plunger is in its second position as before described.

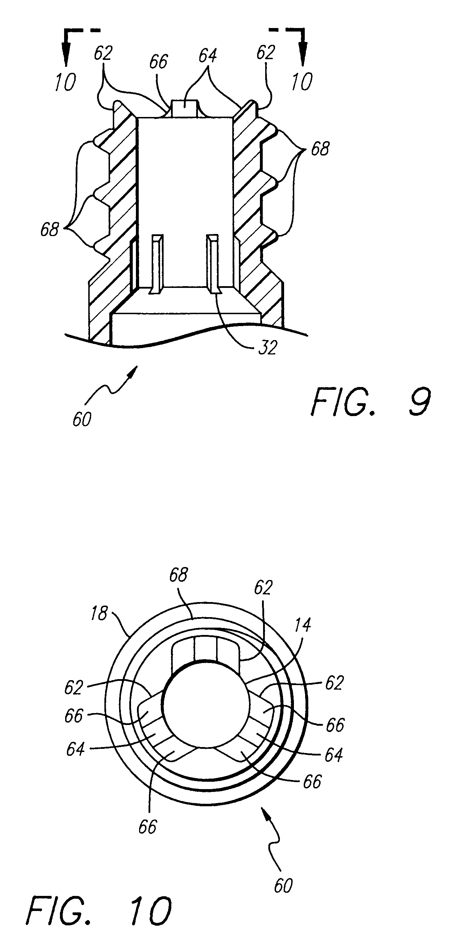

Referring to FIG. 8, a fluid pathway is opened from an inserted male luer fitting 46 to an outlet 29 of the injection site 50 when the inserted male luer fitting moves the plunger 16 to a second position as shown. Withdrawal of the male luer fitting re-seals the injection site as before described in connection with the first embodiment when the plunger returns to its first position as shown in FIG. 7.

Referring to FIGS. 9 and 10, another alternate embodiment of the injection site 60 is illustrated. While functioning otherwise as described in connection with either of the two embodiments of the injection site 10 or 50, further features are added. Three guides 62 are incorporated in the portion of a proximal housing member 18 defining a female luer connection port 14 at a first end of the injection site. The guides are spaced apart at radial angles of one hundred twenty degrees, and each incorporates a surface 64 angled so as to help guide a male luer tip (not shown) into alignment with the connection port as it is being inserted therein. The inclination of the surface 64 on each guide is about forty five degrees in relation to the connection port in the illustrated embodiment. The guides are provided with transitions 66 to eliminate corners where infectious agents or other impurities might accumulate. The transitions also make wiping the injection site incorporating guides 62 prior to connection easier.

Additionally, threads 68 are provided for allowing a compatible luer lock connector (not shown) to be advantageously connected to reduce the risk that the connection will be accidently pulled apart. These threads are an alternative luer lock arrangement to the luer lock portions 15 discussed above in connection with other embodiments.

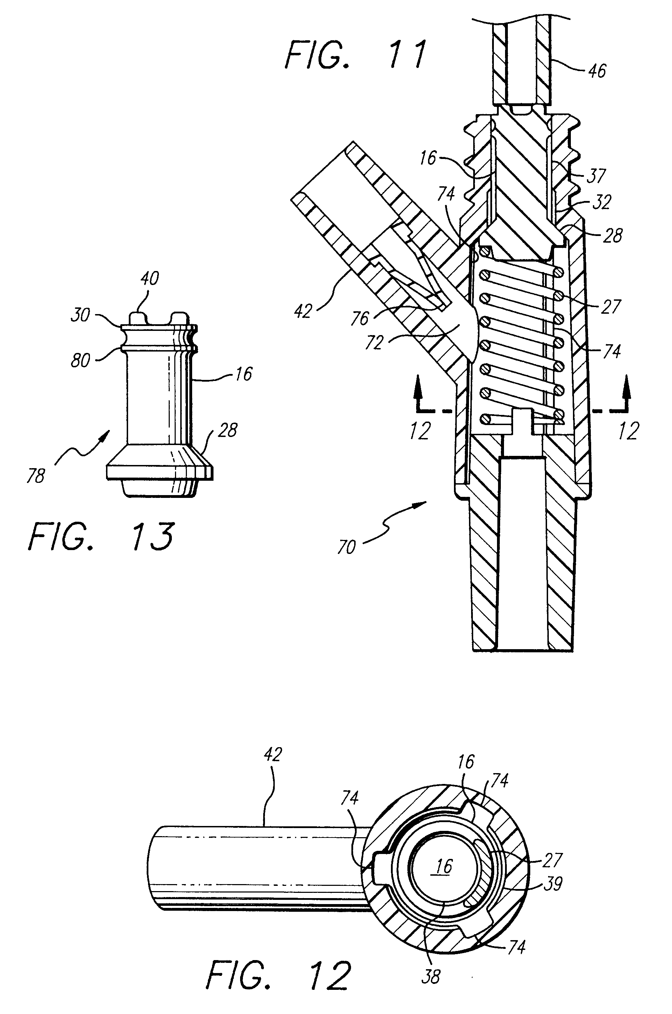

A further alternate embodiment is illustrated in FIGS. 11, 12, and 13. This embodiment incorporates fluid bypass arrangement features from previously described embodiments and adds additional features. As shown in FIGS. 11 and 12 an injection site 70 is configured as the embodiments previously described, but here a fluid bypass is provided by both a side branch port 72, as previously described, and by each of three circumferentially disposed fluid bypass channels 74, which function in a manner similar to the channels 54 before described in connection with the embodiment of the invention in an injection site 50 without a side branch 42. This arrangement incorporating both fluid bypass configurations previously described provides for increased flow capacity through the injection site 70 and has been found to be advantageous in priming the injection site.

In this embodiment, a proximal end of one or more of the channels 74, which end is closest to the connector port 14 at the first end of the injection site 70, is positioned so that upon insertion of a luer tip 46 a small amount of fluid can travel in a proximal direction through the channel 74 around the annular seal portion 28 at the second end of the plunger 16 as the plunger moves into the injection site. This has the effect of equalizing the fluid pressure on either side of the annular seal portion 28. The configuration also allows fluid to pass the annular seal portion at multiple points around the circumference of the plunger, which aides in the breaking up of air bubbles in priming the injection site.

A slight draft, for example one and one-half degrees, may be incorporated in the interior passageway 39 of the housing 18, which makes the injection site easier to manufacture, and also allows increased fluid flow past the annular seal 28 with increased depth of insertion of a luer tip as the diameter of the passageway increases with distance from the connector port 14 at the first end of the housing. Consequently, the depth of the channels 74 may vary with distance from the connection port 14 as less fluid would need to be accommodated with increased distance as more fluid can bypass the annular seal 28 between the seal and the walls of the passageway with increasing distance from the connector port at the first end of the injection site. Employing less draft in the portion of the passageway 39 comprising the channels in comparison with the rest of the passage will provide a variable depth as contemplated.

In a further alternate embodiment, a check valve 76 is incorporated in the side branch 42 (for example a conventional duck-bill valve as shown in FIG. 11) to prevent retrograde flow through the side branch or the secondary line (not shown) attached thereto. Provision of a check valve in the illustrated embodiment prevents fluid from flowing from a primary reservoir (not shown) connected to the injection site 70 by way of the connection port 14 though the side branch 42 to a secondary reservoir (not shown) positioned lower in elevation than the primary reservoir, instead of to the patient as intended.

Referring to FIGS. 11 and 13, in another alternate embodiment of the injection site 70 the plunger 16 incorporates a double annular seal 78 comprising a further annular seal portion 80 of the plunger, in addition to the annular seal portion 30 positioned at the first or proximal end of the plunger described in connection with the embodiments discussed above. Otherwise, the plunger 16 is the same as described previously. The double seal provides further protection against air or fluid leakage as well as the entrance of infectious agents into the injection site.

As with the previously described embodiments, fluid flow through the injection site 70 is prevented until the annular seal 30 at the proximal end of the plunger 16 is moved to a position adjacent fluid bypass openings 32 in the distal portion of the luer well 37. Fluid flow is also accommodated around the further annular seal portion 80 of the plunger through fluid bypass 32, and around the second annular seal portion 28 of the plunger by way of the side branch port 72 and through the circumferentially disposed bypass channels 74 in the manner before described when the plunger is in this second position. Return of the plunger to a first position at the proximal end of the injection site 70, typically by urging of a spring 27 upon removal of a luer tip 46, sealingly closes the injection site as before described.

From the above, it is evident that the present invention provides an advantageous needleless injection site 10, 50 constructed of relatively few parts, which is intuitive to use, simple and reliable in operation, and can be manufactured at a relatively low cost due to the features described above. At the same time, an injection site in accordance with the invention allows safe and convenient handing and infusion of IV fluids in preparing medications and the treatment of patients without use of sharp cannulas. The injection site is convenient to clean, and does not require a protective cap. The design minimizes unwanted fluid and air leakage. The injection site of the invention provides reliable sterile connections without use of a sharp needle thereby reducing accidental needle punctures in connection with patient care.

While several particular forms of the invention have been illustrated and described, it also will be appreciated that various modifications can be made to the present invention without departing from the spirit and scope thereof. It is intended that the invention not be limited, except by the appended claims.

The features disclosed in the foregoing description, in the following claims and/or in the accompanying drawings may, both separately and in any combination thereof, be material for realising the invention in diverse forms thereof.

## Claims

1. An injection site adapted to receive a blunt male fluid connector fitting having diameter and taper parameters within predetermined ranges, to facilitate fluid flow through the injection site without use of a sharp cannula, comprising:
a housing having a first end defining a circular connection port of selected size and a second end, and a passageway therebetween allowing fluid communication between said first and second ends, and a fluid bypass in fluid communication with the passageway intermediate said ends, the fluid bypass being defined by an opening in the wall of the passageway and a fluid pathway disposed radially outward of said passageway in the housing, the opening defining the bypass being placed at a predetermined distance from the first end of the housing between the first and second ends, and having a predetermined length;
an elastomeric plunger, having a first end and a second end, the plunger being movably disposed in the passageway within said housing, having an annular seal portion disposed thereabout between the first and second ends of said plunger, and having a first position adjacent the first end of the housing, wherein the annular seal portion is in sealing contact with the housing, and a second position within the housing where the annular seal portion is located adjacent the opening of said bypass, the connection port being sized and the bypass being placed so that male fittings having said parameters within said ranges will cause the annular seal portion of the plunger to be so located when inserted into the connection port;
biasing means for biasing said plunger toward said first position at the first end of said housing and into sealing contact with said housing; whereby fluid is prevented from flowing through the connector port and passageway when the plunger is in said first position, and fluid is allowed to flow through the connector port and passageway when the plunger is in said second position, fluid flowing through said injection site passing by said seal portion of the plunger by flowing around the seal portion through said fluid bypass.

2. The needleless injection site of claim 1, further comprising a further annular seal portion disposed about the first end of said plunger, said further seal portion being adapted to seal the connection port at the first end of said injection site.

3. The needleless injection site of claim 2, wherein the further annular seal portion disposed about the first end of the plunger is formed of two annular raised portions positioned in close proximity to each other, forming a double annular seal at the first end of said plunger.

4. The needleless injection site of claim 2, further comprising a further fluid bypass incorporated in said housing member which allows fluid flow around said further seal portion when the plunger is in said second position.

5. The needleless injection site of claim 1, further comprising raised ribs incorporated in the housing adjacent the second end, the biasing means being carried by said raised ribs which allow fluid flow around the bottom of the biasing means, thereby facilitating fluid flow through said injection site.

6. The needleless injection site of claim 1, further comprising a plurality of raised guides which incorporate inclined surfaces which assist in guiding a luer tip into said connector port, said guide being disposed about the connector port at the proximal end of the housing.

7. The needleless injection site of claim 1 wherein the fluid bypass comprises at least one channel formed in the inside surface of said passageway, the channel being configured to provide a fluid pathway from a beginning point a selected distance from the first end of the housing toward said second end, extending at least a distance sufficient to allow fluid flow around the annular seal portion and past the plunger when the plunger is in the second position.

8. An injection site adapted to receive a blunt male fluid connector fitting having diameter and taper parameters within selected ranges, to facilitate infusion and withdrawal of fluid through the injection site without use of a sharp cannula, comprising:
a housing having a first end defining a female connection port and a distal end and a passageway therebetween allowing fluid communication between the first end and second ends, a fluid bypass defined by a fluid pathway of selected length disposed radially outward of the inner walls of the housing defining said passageway and opening into the passageway intermediate the ends of the housing a selected distance from the first end, and a further fluid bypass defined by a fluid pathway incorporated in said female connection port disposed radially outward from an inside surface of said connection port and opening into the connection port a selected distance from said first end;
an elastomeric plunger, having a first end and a second end, movably disposed within said housing, the first end thereof being configured to provide a fluid flow pathway between the plunger and an inserted blunt male cannula, said plunger having an annular seal portion at the first end, and an annular seal portion intermediate the first and second ends, and a first position in sealing contact with the housing at the first end thereof, and a second position within the passageway of said housing at which the annular seal portion the first end of the plunger is located adjacent the fluid bypass in the connector port at the first end of the housing and the annular seal portion located between the first end and the second end of the plunger is located adjacent the fluid bypass opening located between the first and second ends of the housing;
biasing means for biasing the plunger to said first position at the first end of said housing and into sealing contact therewith; whereby fluid flow through the injection site is prevented when the plunger is in said first position and the first end of said housing defining a female connection port is sealed against contamination by the annular seal portion at the first end of the plunger, and fluid is allowed to flow through said injection site when the plunger is in said second position, fluid passing by the annular seal portion at the first end of the plunger by flowing into the fluid bypass incorporated in the wall of the connector port at the first end of the housing and around the annular seal portion of said plunger at the first end thereof, and fluid passing by the annular seal portion of the plunger disposed between the first and second ends of the plunger by flowing into the fluid bypass embodied in the wall of the passageway between the first and second ends of the housing and around this annular seal portion of the plunger disposed between the first and second ends thereof, the distance from the first end of the housing to each of the fluid bypass openings and the diameter of the connection port being chosen so as to allow the annular seal portions of said plunger to be adjacent the respective bypass openings when a male blunt cannula having diameter and taper parameters within said selected ranges is inserted.

9. The needleless injection site of claim 8, wherein the fluid bypass incorporated in the connector at the first end of the housing and the fluid bypass embodied in the passageway between the connector at the first end of the housing and the second end of the housing each comprise a plurality of channels disposed circumferentially about an inner surface of the connector and passageway respectively.

10. A Y-site adapted to receive a blunt male fluid connector fitting to facilitate injection and withdrawal of fluid through said Y-site, and which seals upon removal of said male fitting, comprising:
a housing having a first end and a second end and a passageway therebetween allowing fluid communication between the first end and the second ends, and a fluid bypass comprising a side branch port opening into the passageway intermediate the ends and a portion of a side branch fluid passage, said housing defining a female connector at the first end, and having a further fluid bypass channel embodied in said female connector, and raised ribs disposed radially about the interior of said passageway adjacent the second end thereof allowing fluid flow between said ribs into a central portion of the passageway defining an outlet;
an elastomeric plunger, having a first end and a second end, movably disposed within said housing, having a raised portion at the first end thereof to provide a fluid flow clearance between said plunger and an inserted male fitting, and having an annular seal portion at the first end thereof and another annular seal portion between the first and second ends thereof, said plunger cooperating with said housing so that insertion of a male fitting in the first end of the housing displaces the plunger from a first position sealingly contacting said housing at the first end thereof, to a second position where an annular seal portion is located adjacent said fluid bypass comprising the side branch port, and the annular seal portion at the first end of the plunger is located adjacent the fluid bypass within said female connector portion;
biasing means for biasing said plunger to said first position in sealing contact with the housing at the first end thereof, the biasing means being carried by said raised ribs formed in the housing adjacent the second end thereof; whereby, when said plunger is in said first position, fluid flow through said female connector is prevented and the first end of said housing is sealed against contamination by the annular seal portion at the first end of the plunger, and, when said plunger is in said second position, fluid flow through said female luer connector is accommodated, fluid passing by the annular seal portion of the plunger positioned adjacent the side branch port comprising the fluid bypass by flowing into said side branch port and around said annular seal portion of the plunger, and fluid passing through the female fluid connector passing by the annular seal portion at the first end of the plunger by flowing into the fluid bypass disposed in the female connector portion and around the annular seal portion at the first end of said plunger, the dimensions of the plunger and the female connection port at the first end of the housing and the distances from the first end of the housing to the respective fluid bypass openings being selected so that the annular seal portions of the plunger will be adjacent the respective bypass openings over a range of dimensions defining male fittings commonly used define the male fitting inserted.
